(19) Europäisches Patentamt
European Patent Office
Office européen des brevets

(11) **EP 3 692 898 A1**

(12) **EUROPEAN PATENT APPLICATION**

(43) Date of publication:
**12.08.2020 Bulletin 2020/33**

(21) Application number: **19156321.2**

(22) Date of filing: **11.02.2019**

(51) Int Cl.:
*A61B 5/0205* (2006.01)  *A61B 5/08* (2006.01)
*A61B 5/00* (2006.01)  *A61B 5/113* (2006.01)
*A61B 5/11* (2006.01)  *A61B 5/0476* (2006.01)
*A61B 5/0402* (2006.01)  *H04L 1/06* (2006.01)
*A61B 5/05* (2006.01)  *A61B 5/0496* (2006.01)
*A61B 5/0488* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Nokia Technologies Oy**
**02610 Espoo (FI)**

(72) Inventors:
• **ALLOULAH, Mohammed**
  **Cambridge CB5 8SY (GB)**
• **ALI, Kamran**
  **Lancing, Michigan 48823 (US)**
• **KAWSAR, Fahim**
  **Cambridge CB22 5FT (GB)**

(74) Representative: **Nokia EPO representatives**
**Nokia Technologies Oy**
**Karakaari 7**
**02610 Espoo (FI)**

(54) **SLEEP/MOTION DETERMINATION BASED ON WI-FI SIGNALS**

(57)     An apparatus for determining motion of a user, the apparatus comprising means for: obtaining radio-frequency channel state information associated with a receiver; analysing the radio-frequency channel state information to determine subspace components; identifying from the subspace components a periodic breathing motion body motion from an other than breathing body motion; generating, based on the identifying, at least one of a breathing rate parameter and a limb and/or body motion parameter based on the subspace components.

Figure 1

**Description**

Field

The present application relates to apparatus and methods for sleep/motion determination based on radio frequency signals, for example, but not exclusively for sleep/motion determination based on wi-fi radio frequency signals using machine learning classification.

Background

Sleep is crucial for maintaining well-being. Sleep monitoring and data that quantifies sleep such as habits, quality, and/or longer-term duration and consistency patterns can help diagnose sleep disorders such as insomnia, apnoea and narcolepsy. These disorders are prevalent in the general population, and associated with greater risk of cardiovascular, neurological, psychiatric and other disorders.

Some known sleep profiling technologies require extensive on-body contact in order to reliably measure parameters which can be used to determine sleep (as well as other medical) profiling characteristics. These include Electroencephalography (EEG), Electrooculography (EOG), Electromyography (EMG), Electrocardiography (ECG), and body movement.

Summary

There is provided according to a first aspect an apparatus comprising means for: obtaining radio-frequency channel state information associated with a receiver; analysing the radio-frequency channel state information to determine subspace components; identifying from the subspace components a periodic breathing motion body motion from an other than breathing body motion; generating, based on the identifying, at least one of a breathing rate parameter and a limb and/or body motion parameter based on the subspace components.

The apparatus may be further for: obtaining, based on the at least one of a breathing rate parameter and a limb and/or body motion parameter based on the subspace components, at least one sleep state parameter.

The apparatus may be further for: obtaining at least one further modal sleep stage monitoring signal; and generating a mapping between at least one of a breathing rate parameter value range and a limb and/or body motion parameter value range and a sleep state parameter value range based on the at least one further modal sleep stage monitoring signal, such that the obtaining at least one sleep state parameter may be further based on the mapping.

The means for obtaining the at least one further modal sleep stage monitoring signal comprises at least one of: a motion detection and/or sleep mat monitoring signal; a motion detection radar monitoring signal; a EEG monitoring signal; a ECG monitoring signal; an audio monitoring signal; a video monitoring signal; a ballistocardiology monitoring signal; an in-ear monitoring signal; and an inertial sensor signal.

The means for generating a mapping between at least one of a breathing rate parameter value range and a limb and/or body motion parameter value range and a sleep state parameter value range based on the at least one further modal sleep stage monitoring signal may be further for at least one of: generating a machine learning trained mapping; and generating an artificial intelligence trained mapping.

The means for analysing the radio-frequency channel state information to determine subspace components may be further for determining a first order difference between radio-frequency channel state information samples before generating the subspace components.

The means for analysing the radio-frequency channel state information to determine subspace components may be further for low pass filtering radio-frequency channel state information samples before generating the subspace components.

The means for analysing the radio-frequency channel state information to determine subspace components may be further for: performing principle component analysis on the radio-frequency channel state information to determine the subspace components; performing eigenanalysis on the radio-frequency channel state information to determine the subspace components; and performing singular value decomposition analysis on the radio-frequency channel state information to determine the subspace components.

The means for generating, based on the identifying, at least one of a breathing rate parameter and a limb and/or body motion parameter based on the subspace components may be further for identifying the limb and/or body motion parameter based on the subspace components based on: applying a real time hyper-ellipsoidal clustering technique to the subspace components wherein subspace component outliers more than a few sigmas from the mean of a calculated cluster are detected; determining when a number of the detected subspace component outliers passes an event threshold value; mapping the detected subspace component outliers to the limb and/or body motion parameter based on the number of the detected subspace component outliers passing the event threshold value.

The means for identifying from the subspace components a periodic breathing motion body motion from an other than breathing body motion may be further for identifying the other than breathing body motion based on a number of dominant subspace components being greater than a determined threshold.

The means for generating, based on the identifying, at least one of a breathing rate parameter based on the subspace components may be for identifying a breathing rate parameter based on a number of dominant subspace components being less than or equal to the determined threshold.

The means for generating, based on the identifying, at least one of a breathing rate parameter based on the subspace components may be for: bandpass filtering the dominant subspace components; determining the bandpass filtered dominant subspace components have an energy value greater than an outage threshold value; determining a peak motion event within the bandpass filtered dominant subspace components; and mapping the number of peak motion events to a breathing rate parameter value.

The means for determining a peak motion event within the bandpass filtered dominant subspace components may be for: max-min normalising the dominant subspace components; determining a minimum peak prominence (MINPRO), wherein the prominence of a peak determines how much the peak stands out, due to its height and location, relative to other peaks around it; determining a minimum peak distance (MINDIST); determining a minimum peak strength (MINSTR); defining at least one peak motion event based on peaks which have a relative importance of at least MINPRO, and based on a MINDIST selected based on the human respiration rate ranging between 10 to 40 BPM and based on peaks of value greater than MINSTR times a median peak value.

The determined threshold may be 1.

According to a second aspect there is provided a method comprising: obtaining radio-frequency channel state information associated with a receiver; analysing the radio-frequency channel state information to determine subspace components; identifying from the subspace components a periodic breathing motion body motion from an other than breathing body motion; generating, based on the identifying, at least one of a breathing rate parameter and a limb and/or body motion parameter based on the subspace components.

The method may further comprise: obtaining, based on the at least one of a breathing rate parameter and a limb and/or body motion parameter based on the subspace components, at least one sleep state parameter.

The method may further comprise: obtaining at least one further modal sleep stage monitoring signal; and generating a mapping between at least one of a breathing rate parameter value range and a limb and/or body motion parameter value range and a sleep state parameter value range based on the at least one further modal sleep stage monitoring signal, such that the obtaining at least one sleep state parameter may be further based on the mapping.

Obtaining the at least one further modal sleep stage monitoring signal comprises at least one of: obtaining a motion detection and/or sleep mat monitoring signal; obtaining a motion detection radar monitoring signal; obtaining a EEG monitoring signal; obtaining a ECG monitoring signal; obtaining an audio monitoring signal; obtaining a video monitoring signal; obtaining a ballistocardiology monitoring signal; obtaining an in-ear monitoring signal; and obtaining an inertial sensor signal.

Generating a mapping between at least one of a breathing rate parameter value range and a limb and/or body motion parameter value range and a sleep state parameter value range based on the at least one further modal sleep stage monitoring signal may further comprise at least one of: generating a machine learning trained mapping; and generating an artificial intelligence trained mapping.

Analysing the radio-frequency channel state information to determine subspace components may further comprise determining a first order difference between radio-frequency channel state information samples before generating the subspace components.

Analysing the radio-frequency channel state information to determine subspace components may further comprise low pass filtering radio-frequency channel state information samples before generating the subspace components.

Analysing the radio-frequency channel state information to determine subspace components may further comprise: performing principle component analysis on the radio-frequency channel state information to determine the subspace components; performing eigenanalysis on the radio-frequency channel state information to determine the subspace components; and performing singular value decomposition analysis on the radio-frequency channel state information to determine the subspace components.

Generating, based on the identifying, at least one of a breathing rate parameter and a limb and/or body motion parameter based on the subspace components may further comprise identifying the limb and/or body motion parameter based on the subspace components based on: applying a real time hyper-ellipsoidal clustering technique to the subspace components wherein subspace component outliers more than a few sigmas from the mean of a calculated cluster are detected; determining when a number of the detected subspace component outliers passes an event threshold value; mapping the detected subspace component outliers to the limb and/or body motion parameter based on the number of the detected subspace component outliers passing the event threshold value.

Identifying from the subspace components a periodic breathing motion body motion from an other than breathing body motion may further comprise identifying the other than breathing body motion based on a number of dominant subspace components being greater than a determined threshold.

Generating, based on the identifying, at least one of a breathing rate parameter based on the subspace components may further comprise identifying a breathing rate parameter based on a number of dominant subspace components being less than or equal to the determined threshold.

Generating, based on the identifying, at least one of a breathing rate parameter based on the subspace components may further comprise: bandpass filtering the dominant subspace components; determining the bandpass filtered dominant subspace components have an energy value greater than an outage threshold value; determining a peak motion event within the bandpass filtered dominant subspace components; and mapping the number of peak motion events to a breathing rate parameter value.

Determining a peak motion event within the bandpass filtered dominant subspace components may further comprise: max-min normalising the dominant subspace components; determining a minimum peak prominence (MINPRO), wherein the prominence of a peak determines how much the peak stands out, due to its height and location, relative to other peaks around it; determining a minimum peak distance (MINDIST); determining a minimum peak strength (MINSTR); defining at least one peak motion event based on peaks which have a relative importance of at least MINPRO, and based on a MINDIST selected based on the human respiration rate ranging between 10 to 40 BPM and based on peaks of value greater than MINSTR times a median peak value.

The determined threshold may be 1.

According to a third aspect there is provided an apparatus comprising at least one processor and at least one memory including a computer program code, the at least one memory and the computer program code configured to, with the at least one processor, cause the apparatus at least to: obtain radio-frequency channel state information associated with a receiver; analyse the radio-frequency channel state information to determine subspace components; identify from the subspace components a periodic breathing motion body motion from an other than breathing body motion; generate, based on the identifying, at least one of a breathing rate parameter and a limb and/or body motion parameter based on the subspace components.

The apparatus may further be caused to: obtain, based on the at least one of a breathing rate parameter and a limb and/or body motion parameter based on the subspace components, at least one sleep state parameter.

The apparatus may further be caused to: obtain at least one further modal sleep stage monitoring signal; and generate a mapping between at least one of a breathing rate parameter value range and a limb and/or body motion parameter value range and a sleep state parameter value range based on the at least one further modal sleep stage monitoring signal, such that the obtaining at least one sleep state parameter may be further based on the mapping.

Obtaining the at least one further modal sleep stage monitoring signal may cause the apparatus to perform at least one of: obtain a motion detection and/or sleep mat monitoring signal; obtain a motion detection radar monitoring signal; obtain a EEG monitoring signal; obtain a ECG monitoring signal; obtain an audio monitoring signal; obtain a video monitoring signal; obtaining a ballistocardiology monitoring signal; obtain an in-ear monitoring signal; and obtain an inertial sensor signal.

Generating a mapping between at least one of a breathing rate parameter value range and a limb and/or body motion parameter value range and a sleep state parameter value range based on the at least one further modal sleep stage monitoring signal may further cause the apparatus to perform at least one of: generate a machine learning trained mapping; and generate an artificial intelligence trained mapping.

Analysing the radio-frequency channel state information to determine subspace components may further cause the apparatus to determine a first order difference between radio-frequency channel state information samples before generating the subspace components.

Analysing the radio-frequency channel state information to determine subspace components may further cause the apparatus to low pass filter radio-frequency channel state information samples before generating the subspace components.

Analysing the radio-frequency channel state information to determine subspace components may further cause the apparatus to: perform principle component analysis on the radio-frequency channel state information to determine the subspace components; performing eigenanalysis on the radio-frequency channel state information to determine the subspace components; and perform singular value decomposition analysis on the radio-frequency channel state information to determine the subspace components.

Generating, based on the identifying, at least one of a breathing rate parameter and a limb and/or body motion parameter based on the subspace components may further cause the apparatus to identify the limb and/or body motion parameter based on the subspace components based on: applying a real time hyper-ellipsoidal clustering technique to the subspace components wherein subspace component outliers more than a few sigmas from the mean of a calculated cluster are detected; determining when a number of the detected subspace component outliers passes an event threshold value; mapping the detected subspace component outliers to the limb and/or body motion parameter based on the number of the detected subspace component outliers passing the event threshold value.

Identifying from the subspace components a periodic breathing motion body motion from an other than breathing body motion may further be caused to identify the other than breathing body motion based on a number of dominant subspace components being greater than a determined threshold.

Generating, based on the identifying, at least one of a breathing rate parameter based on the subspace components may further cause the apparatus to identify a breathing rate parameter based on a number of dominant subspace components being less than or equal to the determined threshold.

Generating, based on the identifying, at least one of a breathing rate parameter based on the subspace components may further cause the apparatus to: bandpass filter the dominant subspace components; determining the bandpass filtered dominant subspace components have an energy value greater than an outage threshold value; determine a peak motion event within the bandpass filtered dominant subspace components; and map the number of peak motion events to a breathing rate parameter value.

Determining a peak motion event within the bandpass filtered dominant subspace components may further cause the apparatus to: max-min normalise the dominant subspace components; determine a minimum peak prominence (MINPRO), wherein the prominence of a peak determines how much the peak stands out, due to its height and location, relative to other peaks around it; determining a minimum peak distance (MINDIST); determine a minimum peak strength (MINSTR); define at least one peak motion event based on peaks which have a relative importance of at least MINPRO, and based on a MINDIST selected based on the human respiration rate ranging between 10 to 40 BPM and based on peaks of value greater than MINSTR times a median peak value.

The determined threshold may be 1.

According to a fourth aspect there is provided an apparatus comprising obtaining circuitry configured to obtain radio-frequency channel state information associated with a receiver; analysing circuitry configured to analyse the radio-frequency channel state information to determine subspace components; identifying circuitry configured to identify from the subspace components a periodic breathing motion body motion from an other than breathing body motion; generating circuitry configured to generate, based on the identifying, at least one of a breathing rate parameter and a limb and/or body motion parameter based on the subspace components.

According to a fifth aspect there is provided a computer program comprising instructions [or a computer readable medium comprising program instructions] for causing an apparatus to perform at least the following: obtaining radio-frequency channel state information associated with a receiver; analysing the radio-frequency channel state information to determine subspace components; identifying from the subspace components a periodic breathing motion body motion from an other than breathing body motion; generating, based on the identifying, at least one of a breathing rate parameter and a limb and/or body motion parameter based on the subspace components.

According to an sixth aspect there is provided a non-transitory computer readable medium comprising program instructions for causing an apparatus to perform at least the following: obtaining radio-frequency channel state information associated with a receiver; analysing the radio-frequency channel state information to determine subspace components; identifying from the subspace components a periodic breathing motion body motion from an other than breathing body motion; generating, based on the identifying, at least one of a breathing rate parameter and a limb and/or body motion parameter based on the subspace components.

According to a seventh aspect there is provided an apparatus comprising: means for obtaining radio-frequency channel state information associated with a receiver; means for analysing the radio-frequency channel state information to determine subspace components; means for identifying from the subspace components a periodic breathing motion body motion from an other than breathing body motion; means for generating, based on the identifying, at least one of a breathing rate parameter and a limb and/or body motion parameter based on the subspace components.

According to an eighth aspect there is provided a computer readable medium comprising program instructions for causing an apparatus to perform at least the following: obtaining radio-frequency channel state information associated with a receiver; analysing the radio-frequency channel state information to determine subspace components; identifying from the subspace components a periodic breathing motion body motion from an other than breathing body motion; generating, based on the identifying, at least one of a breathing rate parameter and a limb and/or body motion parameter based on the subspace components.

An apparatus comprising means for performing the actions of the method as described above.

An apparatus configured to perform the actions of the method as described above.

A computer program comprising program instructions for causing a computer to perform the method as described above.

A computer program product stored on a medium may cause an apparatus to perform the method as described herein.

An electronic device may comprise apparatus as described herein.

A chipset may comprise apparatus as described herein.

Embodiments of the present application aim to address problems associated with the state of the art.

Summary of the Figures

For a better understanding of the present application, reference will now be made by way of example to the accompanying drawings in which:

Figure 1 shows schematically a system of apparatus suitable for implementing some embodiments;
Figure 2 shows a flow diagram of the operation of the system as shown in Figure 1 in some embodiments;
Figure 3 shows schematically a further system of apparatus suitable for implementing some embodiments;
Figure 4 shows a further flow diagram of the operation of the system as shown in Figure 3 in some embodiments;
Figures 5 and 6 show graphs of simulated sleep/motion estimation using apparatus suitable for implementing some embodiments as shown in Figures 1 and 3; and
Figure 7 shows an example device suitable for implementing the apparatus shown in the figures above.

Embodiments of the Application

The following describes in further detail a suitable system of apparatus and possible mechanisms for the provision of efficient sleep tracking.

As discussed previously several contact and wireless sleep monitoring techniques have been proposed in the past. These have used different sensing modalities, such as in-ear sensing, inertial sensors (Actigraphy), EEG, Audio, Video, Bed sensors (e.g. Ballistocardiography (BCG), pressure and/or motion sensors based techniques) and RF sensing based techniques (e.g. mm-wave, Frequency Modulated Continuous Wave (FMCW) radar, Pulse-Doppler radar, RFID and WiFi based techniques).

The proposals as discussed herein show examples which may demonstrate improvements over existing apparatus and methods. For example some examples as discussed hereafter may not be deemed as privacy invasive as the audio based techniques which record audio during sleep. Additionally the examples as shown herein do not require dedicated radio-frequency hardware and spectrum, and thus avoid adding cost and/or RF regulation hurdles.

Additionally the examples as discussed herein describe apparatus and methods where WiFi signals based sensing provides a low-cost and easily adoptable long-term sleep monitoring system. The apparatus benefit from an environment within which the widespread use of WiFi capable devices (e.g. smart-home assistants, smartphones, etc.) has made WiFi signals the most ubiquitous form of sensing in homes. In such an environment the apparatus as described herein requires no additional transmitter hardware costs.

The examples as discussed here show a WiFi CSI based sleep monitoring approach which is capable of tracking vital signs in an environment- and person-independent manner, for example while the user sleeps comfortably in their bed at home.

The embodiments as described hereafter, show apparatus and methods for obtaining or determining breathing rates and limb/body motions based on analysis of the WiFi signals to generate subspace components. WiFi apparatus implement MIMO (multiple-input multiple-output) and OFDM (orthogonal frequency division multiplexing) where WiFi devices use multiple frequency subcarriers and transmit/receive antennas for data communication. While a person within the environment is sleeping, the subspaces along both frequency (i.e. different OFDM subcarriers) and spatial dimensions (i.e. different transmit/receive antennas) can be affected by breathing, body/limb motion, etc.

As discussed hereafter in further detail when there is no substantial body/limb motion, or where the body/limb movement is periodic breathing movement controlled by the autonomous nervous system there may be only one dominant time-varying component (or a small number of dominant time-varying components) in the subspace. This or these time-varying components can corresponds to defining breathing motion and breathing rates, pulse rate etc. When there is substantial or body/limb motion other than breathing, for example body or limb motion that is that controlled by the somatic nervous system associated with the voluntary control of body movements through the skeletal muscles (such as rolling over, periodic limb movement, or even wakeful movement to fetch a drink or attend the toilet) or non-periodic movement controlled by the autonomous nervous system (such as hypnagogic jerking or twitching), more components along both frequency and spatial dimensions may be present. These subspace components and can be determined and characterized using mathematical analysis such as principal component analysis (PCA) and subspace clustering. In the embodiments as discussed herein there are shown apparatus and methods which, based on this observation, can differentiate between breathing and limb motion without requiring environment-dependent calibrations.

Furthermore as discussed herein in some embodiments further sensing based (cross-modal) supervision sleep stage monitoring may be implemented.

As multi-stage sleep monitoring (i.e. light sleep, deep sleep, rapid-eye-movement sleep, and awake periods) can require significant medical expertise in order to devise the right features, the embodiments may use a cross-modal supervision-based technique, which can obtain and use sleep-stage and breathing data from commercial products (of proven medical-grade accuracies). The data may then be used to train effective WiFi featurisation for sleep stage monitoring and classification with high accuracies.

To this end in some embodiments (multiple) commercial devices, such as radar, sleep pads, Polysomnography (PSG - sleep monitoring gold standard), other breath monitors, and/or sleep stage monitoring devices can be employed to assist the training of systems for sleep stage characterization using the WiFi techniques described herein.

For example in some embodiments the apparatus comprises a data collection system for capturing three sensing modalities concurrently modulated by sleep: (1) WiFi channel variations, (2) signals from a radar-based breath tracking device, and (3) a sleep pad-based sleep stage monitor.

While a user is sleeping, the apparatus can be configured to gather data from these three orthogonal sensing modalities. Deep learning-based architectures for cross-model supervision may be then applied in order to enable off-the-shelf WiFi signals to be capable of sleep stage monitoring and classification.

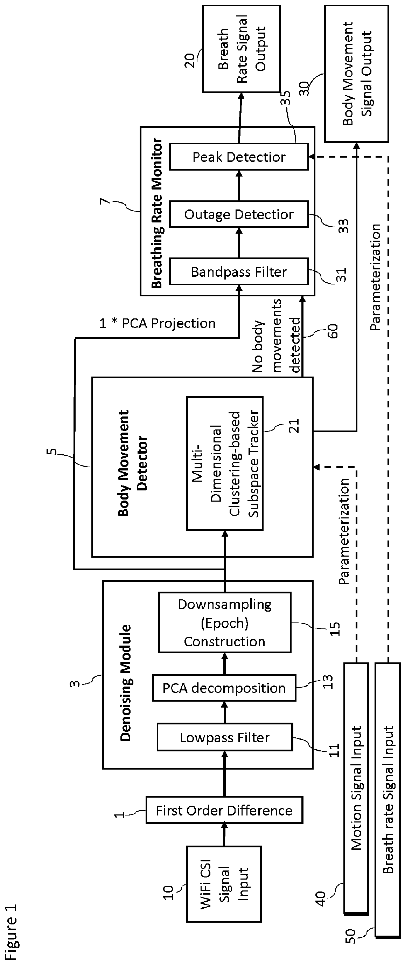

With respect to Figure 1 is shown example apparatus suitable for enabling the operation of the concept. The apparatus comprises a WiFi signal input 10. The WiFi signal input may be configured to receive Wi-Fi CSI signals. In some embodiments this could be implemented by a suitable WiFi receiver generating the CSI data. In some embodiments the input is configured to receive the WiFi CSI data from a physically separate WiFi receiver.

In some embodiments the apparatus comprises a first order difference determiner 1. The first order difference determiner 1 is configured to receive the WiFi CSI data and generate a series of first order differences of the WiFi CSI values.

In some embodiments the apparatus comprises a denoising module 3. WiFi network interface controllers (NICs) generally report noisy channel state information (CSI) values. This is due to hardware limitations (such as slow resolution analogue to digital converters) and changing transmission power and rates. The denoising module 3 is configured to attempt to reduce the noise by a combination of filtering and subspace signal processing.

The denoising module 3 is configured to receive the first order difference values of the WiFi CSI values and generate a series of subspace values for body movement and breathing rate determination. This can be achieved using any suitable mathematical analysis to generate subspaces. In these examples the signals are analysed using a principal component analysis (PCA) decomposition technique. However in other embodiments the sub-spaces can be generated using suitable Eigenvalue analysis (Eigenanalysis) or singular value decomposition (SVD).

In the example shown in Figure 1 the denoising module 3 comprises a lowpass filter 11. As movement during sleep low frequency causes variations which may be typically under 5 Hz the lowpass filter 11 is configured to receive the WiFi difference signals and lowpass filter these signals to separate out the motion related signals from the high frequency noise. Furthermore in some embodiments a further combination of a median filter and an exponential moving average filter can be used to attempt to remove bursty noise and spikes in the CSI signal prior to the lowpass filter. In some embodiments the lowpass filter is a Butterworth lowpass filter. Due to maximally flat amplitude response of Butterworth filter, the application of the filter to the CSI time series does not distort the shape of CSI variations due to body motion.

In some embodiments the CSI values are sampled at a nominal frequency of $F_s$= 800 Hz. The filter cut-off frequency $\omega = 2\pi*f/F_s$ =0.0125$\pi$ rad/s for Butterworth filtering. The same filter can be applied to the WiFi CSI signals of all the subcarriers making sure that every CSI stream experiences the same phase distortion and group delay introduced by the filter. It has been observed that the filtered WiFi signals may retain some noise variations which are not related to activity/breathing. The embodiments descried herein may avoid any further lowpass filtering on CSI streams as it can lead to loss in detail of variations due to activity/breathing behaviour.

In some moments the denoising module 3 comprises a PCA decomposition module 13. The PCA decomposition module 13 can be configured to receive the output of the lowpass filter 11 and performs principal component analysis (PCA) decomposition on the low pass filtered Wi-Fi signal values. As discussed above in some embodiments the PCA decomposition module 13 is replaced by a suitable sub-space generator such as a SVD or Eigenanalysis module. The PCA decomposition module 13 in some embodiments attempts to remove the remaining non activity/breathing behaviour related by utilising the fact that the CSI variations in CSI streams of multiple subcarriers in each Tx-Rx antenna pair are correlated. By applying PCA on CSI values obtained from all carriers and all transmitter-receiver (Tx-Rx) pairs and retain only the waveforms that represent the most common variations to all the subcarriers, i.e. the variations due to breathing and/or body movements during sleep.

That is, signal subspace based filtering enables embodiments to obtain the signals that are representative of the monitored vital signs. PCA (or sub-space analysis in general) also reduces the dimensionality of the data by discarding the principal components unrelated to vital signs (in other words identifying the noise subspaces).

Furthermore in some embodiments of the denoising module 3 comprises a downsampling module 15. The downsampling module 15 is configured to receive the output of the PCA decomposition module 13 and generate downsampled versions of the subspaces. In some embodiments this may be by rearranging the multidimensional filtered CSI data corresponding to each 30 second (or other suitable length) sleep epoch or period into consistent length samples (for example 600 samples per epoch or period) by downsampling and performing zero padding were necessary. These may then be concatenated for real-time tracking of vital signs as shown hereafter.

In some embodiments the apparatus comprises a body movement detector 5. The body movement detector 5 is configured to receive the output of the denoising module 3 (or as shown in Figure 1 the downsampling module 15 within the denoising module 3) subspace values. In some embodiments the body movement detector comprises a multidimensional clustering based subspace tracker 21. The multidimensional clustering based subspace tracker 21 is configured to receive the subspace values and determine whether or not there is body movement and the degree of body movement.

In some embodiments the multidimensional clustering based subspace tracker 21 is configured to determine and track body movement by determining the number of dominant subspaces within the subspace values. Where the number of dominant subspaces is greater than a threshold value then body movement is determined. In some moments this threshold value is a single dominant subspace. In other words if the output of the denoising module 3 has more than one dominant subspace then the body movement detector determines that there is significant limb/body movement (in other words body movement other than breathing). Furthermore these subspace signals can be used to determine and track body movement. In such embodiments a body movement signal 30 can be output which provides a suitable parameter value identifying the motion of the limbs and/or body. Furthermore in some embodiments an indicator (which may be a binary indicator) of whether or not body movement has been detected can also be output such as "no body movements detected" signal 60 from the body movement detector 5 to the breathing rate monitor 7.

As discussed above the MIMO (multiple input multiple output) and OFDM (orthogonal frequency division multiplexing) based WiFi use multiple frequency subcarriers and Tx-Rx (transmitter-receiver) antennas for data communication. The MIMO system between the Tx-Rx antennas and the OFDM subcarriers form a multidimensional tensor along space-frequency axes. Contained in the tensor is the signal subspace to be tracked for respiration and body motion estimation. As discussed while a user sleeping, the subspace along the frequency and spatial dimensions is affected by both breathing and body/limb motion. When there is no body/limb motion other than breathing, there is only one (or a small number of) dominant, time-varying component of subspace, which corresponds to respiration. PCA can be used to sort the different principal components into a descending order of their variation. During sleep the signal subspace may be quiet and respiration is captured in the top (or dominant) PCA projection of the WiFi CSI time series. However during episodes of other body movements, for example during roll overs or arm/leg movement, more signal subspace components evolve, since body movements cause more pronounced variations in the spatial-frequency subspace compared to breathing movements. Therefore in some embodiments a multidimensional hyper-ellipsoid clustering on a portion of the signal subspace of CSI time series is employed in order to robustly distinguish body activity/limb movement from respiration.

To robustly distinguish body movements from respiration a multidimensional anomaly detection approach may be employed where a real time hyper-ellipsoidal clustering technique is used to detect body/limb motion events in CSI time series. At a high level this clustering is a high-dimensional generalisation to a Gaussian outlier rejection technique whereby measurements more than a few sigmas are rejected.

This may mathematically be described based on:

$R_k = \{r_1, r_2, ..., r_k\}$ which are the first k samples of CSI vectors containing values from the selected signal subspace. In some embodiments PCA projections 4 and 5 can be implemented. Each sample $r_i$ is a $d \times 1$ vector in $R^d$ where d is number of signal subspace components. The hyper-ellipsoid technique clusters the normal data points (in other words when there is no body movement in the environment) and any points lying outside the cluster are declared as outliers. The boundary of the cluster (a hyper-ellipsoid in this case) is related to a distance metric which is a function of mean $m_{R,k}$ and covariance $S_k$ of the incoming signal subspace components $R_k$. In some embodiments the Mahalanobis distance metric, $D_i$, is used for which the cluster is arrived at according to the following:

$$e_k(m_R \cdot S_k^{-1} \cdot t) = \left\{ r_i \in R^d \,\middle|\, \sqrt{\left(r_i - m_{R,k}\right)^T S_k^{-1} \left(r_i - m_{R,k}\right)} \le t \right\}$$

where $e_k$ is the set of normal data points whose Mahalanobis distance $D_i < t$ and t is the effective radius of the hyper-ellipsoid. The choice of t depends on the distribution of the normal data points. If the normal data follows a chi-squared distillation it has been shown that up to 98% of the incoming normal data can be enclosed by the boundary of a hyper-ellipsoid, if the effective radius t is chosen such that $t^2 = \left(\chi d^2\right)^{-1}_{0.98}$. Data samples $r_i$ for which $D_i > t$ are therefore identified as outliers. In some embodiments as it is often not practical to store all of the samples of a streaming data a recursive algorithm is used to update $e_k$. Where $r_{k+1}$ be the most recent CSI sample $m_{R,k+1} = \frac{km_{R,k} + r_{k+1}}{k+1}$ and

$$m_{R^2,k+1} = \frac{km_{R^2,k} + r_{k+1}r^T_{k+1}}{k+1}$$ can be updated recursively from the previous means. By substituting covariance matrix $$S_k = m_{R^2,k+1} - \left(m_{R,k}m^T_{R,k}\right)$$ into the $e_k$ equation above the $e_k$ term can be expressed as means. The resulting equation updates the cluster boundary and classifies the incoming data samples as normal readings or outliers. In some embodiments the above can be used for initial estimation of mean and covariance. Afterwards in some embodiments the mean $m_{R,k}$ is recursively calculated using an exponential moving average technique where mean $m_{R,k+1}$ is updated as $m_{R,k+1} = \alpha m_{R,k} + (1 - \alpha)r_{k+1}$ where $\alpha = 0.9995$. Moreover after the initial estimation of covariance in some embodiments the value is recursively updated using the following (which avoids the extra computation required for calculating the inverse of the matrix S):

$$S_{k+1}^{-1} = \frac{kS_k^{-1}}{\alpha(k-1)} \times \left[1 - \frac{(r_{k+!} - m_{R,k})(r_{k+!} - m_{R,k})^T S_k^{-1}}{\frac{(k-1)}{\alpha} + (r_{k+!} - m_{R,k})^T S_k^{-1}(r_{k+!} - m_{R,k})}\right].$$

To determine the start and end of the activity waveforms both cardinality and temporal proximity to the detected outliers are used. If the number of consecutive outliers increases a threshold $E_1$ a micro-event is defined. Multiple micro-events constitute an activity event. All the data points including the points constituting the micro-evens as well as the points between the consecutive micro events are recorded as part of the activity waveform. In some embodiments there can be implemented merging of the micro-events which are within $E_2$ data points of each other. Both $E_1$ and $E_2$ are tuneable thresholds.

In some embodiments the training of the body movement detector 5 (and furthermore the multi-dimensional clustering-based subspace tracker 21) is performed based on a motion signal input 40. The motion signal input can, in some embodiments be used to train or adapt the body movement detector 5 such that there is a mapping between subspace signals and the body movement signal output 30 which is determined based on the motion signal input. In some embodiments the training is a predetermined or generic training. In some embodiments an initial generic training can be further refined by in-situ training.

In some embodiments the apparatus comprises a breathing rate monitor 7. The breathing rate monitor 7 can be configured to receive the primary dominant subspace signal component (for example the first PCA projection) and in some embodiments furthermore an indicator 60 of whether or not there has been any detected body movement (as detected by the body movement detector 5). The breathing rate monitor 7 is configured to determine from the primary subspace signal component the breathing rate when the body movement detector indicates that there is no limb and/or body motion other than breathing motion.

The breathing rate monitor 7 in some embodiments is configured to determine human respiration based on the detection of the motion of the chest and lungs during inspiration (when air is taken into the lungs) and expiration (when air is blown out of the lungs). These motions are often periodic (for example in case of healthy subjects) and therefore cause periodic variations in the WiFi signals which can be extracted using the CSI data. In the absence of other body movements the first PCA protection of the CSI data is used in the embodiments described herein to capture these variations due to breathing. However as the minute variations are often embedded in noise and because the subject may not be in the proximity of the receiver it cannot be assumed that respiration signal exists in a particular sleep epoch or period. Therefore to robustly track the breathing rate the following signal processing pipeline can be implemented in some embodiments.

The breathing rate monitor 7 in some implements comprises a bandpass filter 31. The bandpass filter 31 is configured to receive the first (principal component analysis) PCA projection and bandpass filter the values.

The bandpass filter 31 in some embodiment is configured to extract the periodic variations in the CSI signal due to respiration. In some embodiments the bandpass filter may be implemented as a Butterworth bandpass filter on the first PCA projection. The filtering parameters, in some embodiments, may be set and chosen based on the typical respiration rate of between 10 to 40 breaths per minute (BPM) to attempt to remove any non-breathing related noise present in the signal.

The breathing rate monitor 7 furthermore can comprise an outage detection module 33 configured to receive the output of the bandpass filter 31. The outage detection module 33 is configured to process the bandpass filtered first PCA projection signal to determine any outages. In some embodiments an outage is an event where variations due to respiration are not present in the CSI signal. The outage detection module 33 can thus in some embodiments determine a noise floor of the environment using the first PCA projection. The outage detection module 33 furthermore may perform real-time tracking where the average power of the signal (calculated over a 7.5 second window of a 30 second sleep epoch or period) is determined and compared to a determined noise floor. In some embodiments an outage is determined when the average power for the epoch or period is greater than the noise floor. The breathing rate monitor 7 furthermore is configured not to generate breathing rate estimations when the outages occur.

The breathing rate monitor 7 furthermore may comprise a peak detector 35 configured to receive the output of the outage detector 33. The peak detector 35 is configured to generate a suitable output indicating the breath rate, as a breath rate signal output 20. In some embodiments the peak detector 35 is configured to output or measure respiration rate in terms of breaths per minute (BPM). These may be measured over a window of two sleep epochs or periods in length which moves over concatenated data from multiple consecutive epochs. To report the BPM every second the method moves the window over the concatenated data for each second (in other words over 20 samples a time).

To measure respiration rate the peak detector 35 is configured to max-min normalise the signal so that parameterization can be easily generalized for different users.

Then to robustly detect the number of peaks the method uses 3 parameters: a minimum peak prominence (MINPRO), minimum peak distance (MINDIST) and minimum peak strength (MINSTR). The prominence of a peak determines how much the peak stands out, due to its height and location, relative to other peaks around it. The method is configured to tune MINPRO such that it is configured to detect those peaks which have a relative importance of at least MINPRO. The method tunes MINDIST based on the human respiration rate ranging between 10 to 40 BPM so that redundant peaks are discarded otherwise. The method furthermore may be configured to discard other redundant peaks by choosing peaks of value greater than MINSTR times the median peak value. In some embodiments MINPRO= 0.05, MINDIST = 1.5 seconds and MINSTR = 0.5. These values can be configured during the design of the apparatus and may not require any end user calibration.

In some embodiments the training of the breath rate monitor 7 (and furthermore the peak detector 35) is performed based on a breath rate signal input 50. The breath rate signal input 50, can in some embodiments be used to train or adapt the breath rate monitor 7 such that there is a mapping between the peak detector peak subspace signal value and the breath rate signal output. In some embodiments the training is a predetermined or generic training. In some embodiments an initial generic training can be further refined by in-situ training.

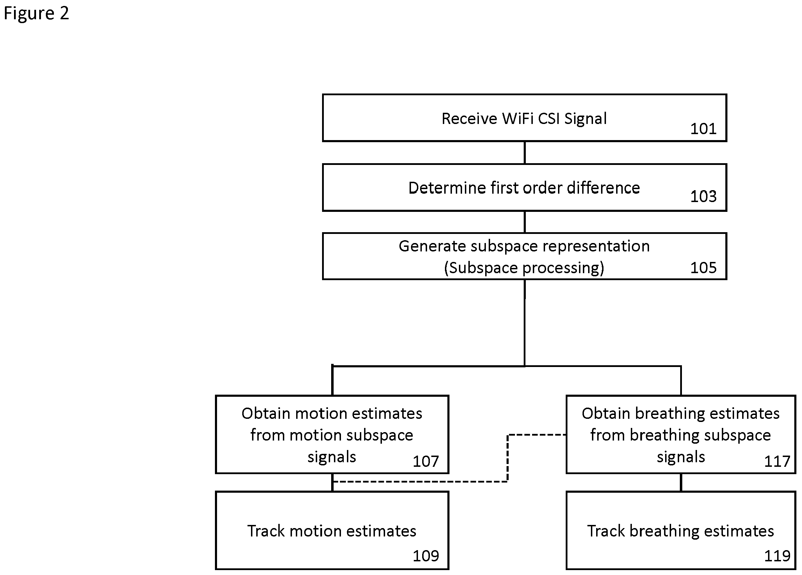

With respect to Figure 2 an example flow diagram of the apparatus shown in Figure 1 is further described.

The first operation is that of receiving the WiFi CSI signal as shown in Figure 2 by step 101.

The following step is that of determining a first order difference as shown in Figure 2 by step 103.

The next operation is generating a subspace representation of the first order difference signal using any suitable subspace processing technique. The generation of the subspace is shown in Figure 2 by step 105.

The next operation is that of obtaining motion estimates from the generated subspace signals. In some embodiments this may comprise determining whether the generated subspace signals have more than the threshold number of dominant subspaces (in other words more than one dominant subspace) and generate a motion detection indicator signal based on this comparison which can be used to determine whether the breathing rate can be determined. In some embodiments the motion estimate is determined based on the dominant subspace values as shown in Figure 2 by step 107.

Having determined a body motion estimate this may then be monitored or tracked as shown in Figure 2 by step 109.

Additionally the system may obtain breathing rate estimates from the generated subspace signals (and further based on a determination of body motion). This for example may be implemented by bandpass filtering the primary dominant subspace value and then mapping the value to a breathing rate as shown in Figure 2 by step 117.

Having determined a breathing rate estimate this may then be monitored or tracked as shown in Figure 2 by step 119.

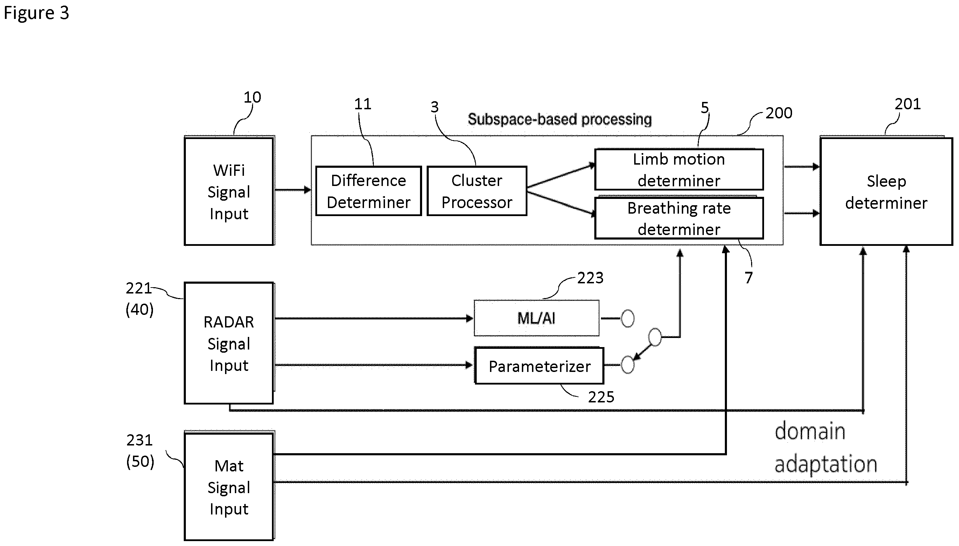

With respect to Figure 3 is shown the apparatus further comprising the sleep determiner 201.

In the example shown in Figure 3 there is shown the WiFi signal input 10 which is passed to the subspace based processor 200 (configured to output the motion and breathing rate signals) which is then output to the sleep determiner 201. The subspace based processor 200 comprises the difference determiner 11, the cluster processor 3 (denoising module comprising the PCA decomposition algorithms) and the limb and/or body movement detector (limb motion determiner) 5 and the breathing rate monitor (breathing rate determiner) 7.

Furthermore there is received a radar signal input 221 (which may be considered to be similar to the motion signal input 40 shown in Figure 1) and mat signal input 231 (which may be considered to be similar to the breath rate signal input 50). These may be passed directly or indirectly to the subspace based processor 200 and the sleep determiner 201. For example as shown in Figure 3 the radar signal input 231 is passed via a machine learning/AI module 223 or parameterizer 225.

Thus domain adaptation machine learning techniques can then be used to arrive at an automatic transformation that relates WiFi-based motion and breathing estimates to sleep stages.

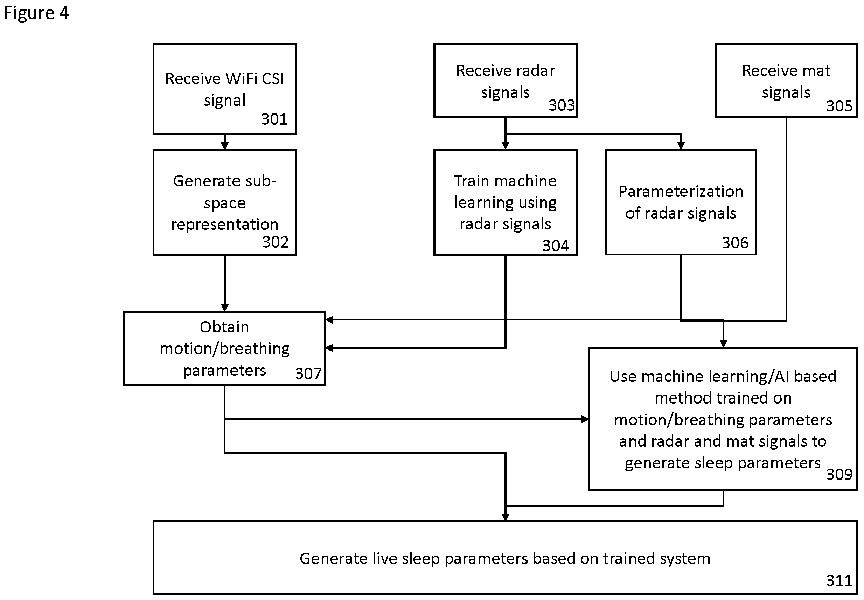

With respect to Figure 4 is shown the operations of the apparatus shown in Figure 3 according to some embodiments.

A first operation may be one of receiving the WiFi CSI signal as shown in Figure 4 by step 301.

Furthermore is received the radar signals as shown in Figure 4 by step 303.

Also is shown the operation of receiving the mat signals (indicating breathing) as shown in Figure 4 by step 305.

The WiFi CSI signals can be used to generate subspace representations as shown in Figure 4 by step 302.

The radar signals may be processed to train machine learning for the assistance of estimation of motion/breathing parameters as shown in Figure 4 by step 304.

The radar signals may also be processed to generate parameters which also assist the estimation of motion/breathing parameters as shown in Figure 4 by step 306.

The radar and mat signals (processed and unprocessed) with the subspace signals can be used to obtain the motion/breathing estimations as shown in Figure 4 by step 307.

Furthermore radar, mat and motion/breathing parameters can be processed, for example by the use of a machine learning/AI algorithm to generate a mapping between motion/breathing parameters and sleep states or sleep parameters as shown in Figure 4 by step 309.

Having generated the trained system then later motion/breathing parameters can be used to determine sleep state values or sleep parameters as shown in Figure 4 by step 311.

In the above examples, a radar module and a sleep mat are depicted. However, in other embodiments domain adaptation machine learning techniques can be used to arrive at an automatic transformation that relates WiFi-based motion and breathing estimates to sleep stages directly based on any suitable supervising modality selection. For example a medical-grade sleep profiler or a wireless device.

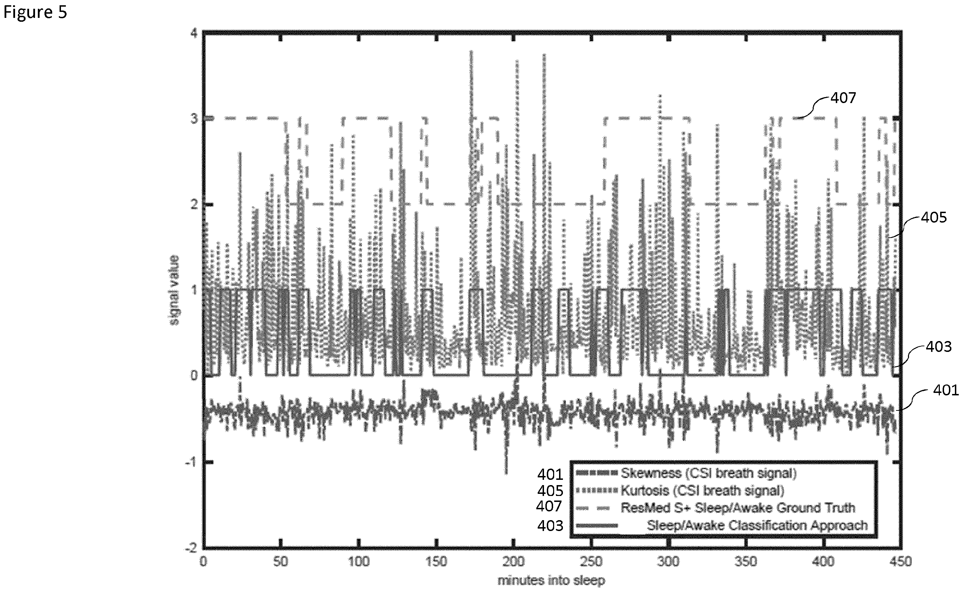

With respect to Figure 5 is shown an example simulation of a skewness breath signal (the thick dashed line which is the lower trace in Figure 5) 401, a kertosis breath signal (the narrow dash line in Figure 5) 405, a medical sleep/awake signal (the spaced dashed line which is the upper of the on/off traces in Figure 5) 407 and the simulated sleep/awake signal (the solid line which is the lower of the on/off traces in Figure 5) 403 based on the WiFi estimation.

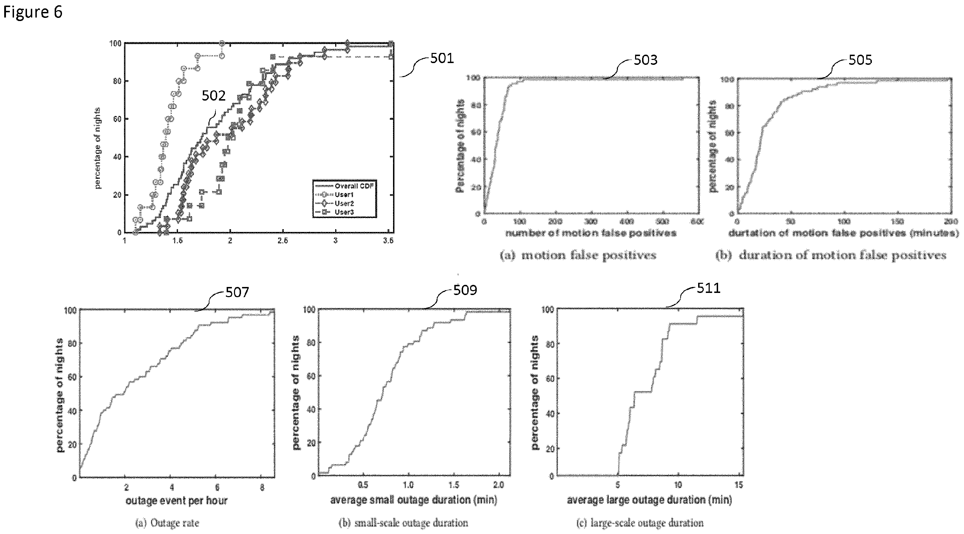

Furthermore with respect to Figure 6 is shown a series of graphs of further simulations including continuous distribution functions of overall and per-user respiration rate MSE as compared to a radar based signal ground truth. In this figure the accuracy of the WiFi-based respiration estimation is compared with the ground truth reported by a dedicated radar-based system. In this example the mean squared error (MSE) of instantaneous (per second) respiration rate estimate per participant and across an entire night of sleep is calculated. The overall cumulative distribution function (CDF) of the MSE error in breath per minute (BPM) is depicted 501. Inspecting the overall curve (the solid line) 502 then on average the respiration estimate as produced by embodiments as discussed herein is accurate to within 1.73 BPM, while its 95th percentile confidence is under 2.8 BPM.

Figure 6 furthermore shows a continuous distribution functions of numbers and durations of motion false positives as compared to a radar based signal ground truths. Radar and WiFi are both very sensitive to body motion. Whenever a user moves in their bed, both the embodiments and known radar system successfully detect the motion event. However there may be scenarios where the system according to some embodiments detected body movement but the (radar system) ground truth remained undisturbed (contained a breathing signal only). These may be defined as motion false positives (MFPs), which can be attributed to other movement present in the environment (e.g. when one of the house-mates wakes up to get water, etc.). To understand how significant such MFPs can be in real-world deployments of the system according to some embodiments, two key metrics are evaluated. These are namely the number and duration of MFPs per night's sleep. Figure 6 shows plots of CDFs of these two metrics 503 and 505 evaluated over more than 65 nights from a database of results. The apparatus according to some embodiments detected less than 76 MFPs occurrences more than 95% of tested nights. Moreover, when MFPs occur, their collective duration remains bounded under 100 minutes more than 93% of the time, and is less than 40 minutes more than 80% of the time. This a minor fraction of an average sleep duration of entire 450+ minutes. Thus the body movement estimation provided by the embodiments may satisfy the body movement detection accuracy requirements expected from an in-home use case sleep monitor.

Furthermore Figure 6 shows second-order statistics of respiration estimation outage events. These plots assess the embodiments ability to supply qualitative breathing estimation in the real-world by measuring the outage performance (i.e. when the embodiments are not able to detect any vital signs).

In wireless propagation literature, two second-order statistics are oftentimes discussed: level crossing rate (LCR), and average fade duration (AFD). LCR and AFD have been extensively studied in body area network (BAN) literature owing to the complex and non-stationary way in which a human body interacts with the wireless channel. Basically, the power fluctuations that cause respiration estimation to fail are analysed. As discussed above the instance of insufficient power in the first PCA projection is defined as an outage event. In this example the second-order temporal statistics of outage events are provided to summarise expectations for the deployability of practical WiFi CSI based sleep monitoring. The statistics derived from the entire dataset which corresponds to > 500 hours of sleep have been shown here.

To detect outage events, the LCR of PCA power is analysed with respect to the noise floor. The rate at which signal quality deteriorates below a certain threshold is designated as the outage rate. One graph 507 depicts such outage rate per hour calculated across the sleep dataset. On average, the respiration estimation of participants experienced 2 outage events per hour. At 95 percentile confidence, outage amounted to less than 6.6 events per hour.

To detect how long the inability to estimate respiration lasts once outage occurs, the average outage duration, which is analogous to AFD, is analysed. However, within the context of sleep monitoring, a further piece of detail should be considered in order to fully appreciate the natural variations in outage duration. Broadly, there are two factors that impact the ability to estimate respiration. These factors can be either small-scale or large-scale. Small-scale outage arises from users turning in bed while sleeping. That is, certain sleep postures may momentarily hamper the ability of the embodiments to detect respiration either due to the transient motion involved, or simply changing the posture in such a way that chest movements are less picked up by WiFi. In contrast, large-scale outage occurs when a user gets out of bed during the night, for instance to fetch water. As discussed earlier, signal subspace tracking allows an inferring as to when big movements mask the much fainter respiration signal. However, it may be desirable to know how such small- and large-scale outage events are distributed naturally in the real-world. To this end, a design threshold has been introduced to separate the two outage events. From the analysis of the dataset, the design threshold is set to 5 minutes. Meaning, an outage event greater than 5 minutes is deemed one likely to have been triggered by a user waking up at night. The graph 509 elaborates on the statistical behaviour of small-scale outage. On average, small-scale outage events lasted for 0.7 minutes, while the 95th percentile confidence outage duration is under 1.62 minutes. CDF of the duration of large-scale outage is shown in graph 511. Large-scale outage duration averaged around 6.38 minutes while its 95th percentile confidence is under 11.56 minutes, although durations in excess of 15 minutes can occur.

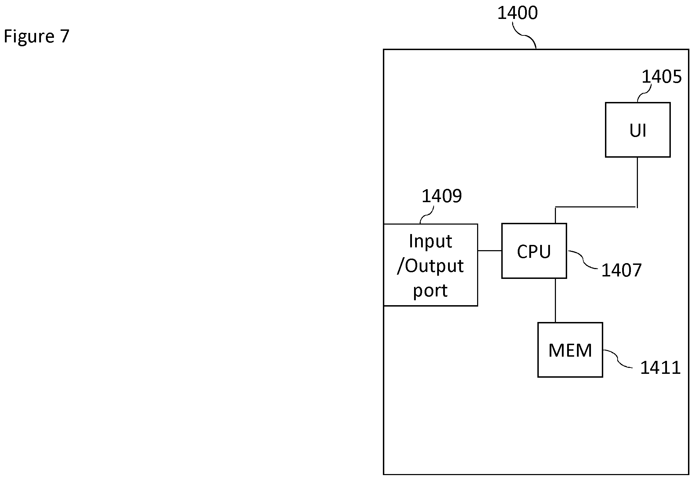

With respect to Figure 7 an example electronic device which may be used as one of the sensors or management system apparatus is shown. The device may be any suitable electronics device or apparatus.

In some embodiments the device 1400 comprises at least one processor or central processing unit 1407. The processor 1407 can be configured to execute various program codes such as the methods such as described herein.

In some embodiments the device 1400 comprises a memory 1411. In some embodiments the at least one processor 1407 is coupled to the memory 1411. The memory 1411 can be any suitable storage means. In some embodiments the memory 1411 comprises a program code section for storing program codes implementable upon the processor 1407. Furthermore in some embodiments the memory 1411 can further comprise a stored data section for storing data, for example data that has been processed or to be processed in accordance with the embodiments as described herein. The implemented program code stored within the program code section and the data stored within the stored data section can be retrieved by the processor 1407 whenever needed via the memory-processor coupling.

In some embodiments the device 1400 comprises a user interface 1405. The user interface 1405 can be coupled in some embodiments to the processor 1407. In some embodiments the processor 1407 can control the operation of the user interface 1405 and receive inputs from the user interface 1405. In some embodiments the user interface 1405 can enable a user to input commands to the device 1400, for example via a keypad/touch interface. In some embodiments the user interface 1405 can enable the user to obtain information from the device 1400. For example the user interface 1405 may comprise a display configured to display information from the device 1400 to the user. The user interface 1405 can in some embodiments comprise a touch screen or touch interface capable of both enabling information to be entered to the device 1400 and further displaying information to the user of the device 1400.

In some embodiments the device 1400 comprises an input/output port 1409. The input/output port 1409 in some embodiments comprises a transceiver. The transceiver in such embodiments can be coupled to the processor 1407 and configured to enable a communication with other apparatus or electronic devices, for example via a wireless communications network. The transceiver or any suitable transceiver or transmitter and/or receiver means can in some embodiments be configured to communicate with other electronic devices or apparatus via a wire or wired coupling.

The transceiver can communicate with further apparatus by any suitable known communications protocol. For example in some embodiments the transceiver can use a suitable universal mobile telecommunications system (UMTS) protocol, a wireless local area network (WLAN) protocol such as for example IEEE 802.X, a suitable short-range radio frequency communication protocol such as Bluetooth, or infrared data communication pathway (IRDA).

The transceiver input/output port 1409 may be configured to receive the signals and in some embodiments determine the parameters as described herein by using the processor 1407 executing suitable code.

In general, the various embodiments of the invention may be implemented in hardware or special purpose circuits, software, logic or any combination thereof. For example, some aspects may be implemented in hardware, while other aspects may be implemented in firmware or software which may be executed by a controller, microprocessor or other computing device, although the invention is not limited thereto. While various aspects of the invention may be illustrated and described as block diagrams, flow charts, or using some other pictorial representation, it is well understood that these blocks, apparatus, systems, techniques or methods described herein may be implemented in, as non-limiting examples, hardware, software, firmware, special purpose circuits or logic, general purpose hardware or controller or other computing devices, or some combination thereof.

As used in this application, the term "circuitry" may refer to one or more or all of the following:

(a) hardware-only circuit implementations (such as implementations in only analog and/or digital circuitry) and
(b) combinations of hardware circuits and software, such as (as applicable):

(i) a combination of analog and/or digital hardware circuit(s) with software/firmware and
(ii) any portions of hardware processor(s) with software (including digital signal processor(s)), software, and memory(ies) that work together to cause an apparatus, such as a mobile phone or server, to perform various functions) and

(c) hardware circuit(s) and or processor(s), such as a microprocessor(s) or a portion of a microprocessor(s), that requires software (e.g., firmware) for operation, but the software may not be present when it is not needed for operation.

The embodiments of this invention may be implemented by computer software executable by a data processor of the mobile device, such as in the processor entity, or by hardware, or by a combination of software and hardware. Further in this regard it should be noted that any blocks of the logic flow as in the Figures may represent program steps, or interconnected logic circuits, blocks and functions, or a combination of program steps and logic circuits, blocks and functions. The software may be stored on such physical media as memory chips, or memory blocks implemented within the processor, magnetic media such as hard disk or floppy disks, and optical media such as for example DVD and the data variants thereof, CD.

The memory may be of any type suitable to the local technical environment and may be implemented using any suitable data storage technology, such as semiconductor-based memory devices, magnetic memory devices and systems, optical memory devices and systems, fixed memory and removable memory. The data processors may be of any type suitable to the local technical environment, and may include one or more of general purpose computers, special purpose computers, microprocessors, digital signal processors (DSPs), application specific integrated circuits (ASIC), gate level circuits and processors based on multi-core processor architecture, as non-limiting examples.

Embodiments of the inventions may be practiced in various components such as integrated circuit modules. The design of integrated circuits is by and large a highly automated process. Complex and powerful software tools are available for converting a logic level design into a semiconductor circuit design ready to be etched and formed on a semiconductor substrate.

Programs, such as those provided by Synopsys, Inc. of Mountain View, California and Cadence Design, of San Jose, California automatically route conductors and locate components on a semiconductor chip using well established rules of design as well as libraries of pre-stored design modules. Once the design for a semiconductor circuit has been completed, the resultant design, in a standardized electronic format (e.g., Opus, GDSII, or the like) may be transmitted to a semiconductor fabrication facility or "fab" for fabrication.

The foregoing description has provided by way of exemplary and non-limiting examples a full and informative description of the exemplary embodiment of this invention. However, various modifications and adaptations may become apparent to those skilled in the relevant arts in view of the foregoing description, when read in conjunction with the accompanying drawings and the appended claims. However, all such and similar modifications of the teachings of this invention will still fall within the scope of this invention as defined in the appended claims.

**Claims**

1. An apparatus for determining motion of a user, the apparatus comprising means for:

obtaining radio-frequency channel state information associated with a receiver;
analysing the radio-frequency channel state information to determine subspace components;
identifying from the subspace components a periodic breathing motion body motion from an other than breathing body motion;
generating, based on the identifying, at least one of a breathing rate parameter and a limb and/or body motion parameter based on the subspace components.

2. The apparatus as claimed in claim 1, further for:
obtaining, based on the at least one of a breathing rate parameter and a limb and/or body motion parameter based on the subspace components, at least one sleep state parameter.

3. The apparatus as claimed in claim 2, further for:

obtaining at least one further modal sleep stage monitoring signal; and
generating a mapping between at least one of a breathing rate parameter value range and a limb and/or body motion parameter value range and a sleep state parameter value range based on the at least one further modal sleep stage monitoring signal, such that the obtaining at least one sleep state parameter is further based on the mapping.

4. The apparatus as claimed in claim 3, wherein the means for obtaining the at least one further modal sleep stage monitoring signal comprises at least one of:

a motion detection and/or sleep mat monitoring signal;
a motion detection radar monitoring signal;
a EEG monitoring signal;
a ECG monitoring signal;
an audio monitoring signal;
a video monitoring signal;
a ballistocardiology monitoring signal;
an in-ear monitoring signal; and
an inertial sensor signal.

5. The apparatus as claimed in any of claims 3 and 4, wherein the means for generating a mapping between at least one of a breathing rate parameter value range and a limb and/or body motion parameter value range and a sleep state parameter value range based on the at least one further modal sleep stage monitoring signal is further for at least one of:

generating a machine learning trained mapping; and
generating an artificial intelligence trained mapping.

6. The apparatus as claimed in any of claims 1 to 5, wherein the means for analysing the radio-frequency channel state information to determine subspace components is further for determining a first order difference between radio-frequency channel state information samples before generating the subspace components.

7. The apparatus as claimed in any of claims 1 to 6, wherein the means for analysing the radio-frequency channel state information to determine subspace components is further for low pass filtering radio-frequency channel state information samples before generating the subspace components.

8. The apparatus as claimed in any of claims 1 to 7, wherein the means for analysing the radio-frequency channel state information to determine subspace components is further for:

performing principle component analysis on the radio-frequency channel state information to determine the subspace components;
performing eigenanalysis on the radio-frequency channel state information to determine the subspace components; and
performing singular value decomposition analysis on the radio-frequency channel state information to determine the subspace components.

9. The apparatus as claimed in any of claims 1 to 8, wherein the means for generating, based on the identifying, at least one of a breathing rate parameter and a limb and/or body motion parameter based on the subspace components is further for identifying the limb and/or body motion parameter based on the subspace components based on:

applying a real time hyper-ellipsoidal clustering technique to the subspace components wherein subspace component outliers more than a few sigmas from the mean of a calculated cluster are detected;
determining when a number of the detected subspace component outliers passes an event threshold value;
mapping the detected subspace component outliers to the limb and/or body motion parameter based on the number of the detected subspace component outliers passing the event threshold value.

10. The apparatus as claimed in any of claims 1 to 9, wherein the means for identifying from the subspace components a periodic breathing motion body motion from an other than breathing body motion is further for identifying the other than breathing body motion based on a number of dominant subspace components being greater than a determined threshold.

11. The apparatus as claimed in claim 10, wherein the means for generating, based on the identifying, at least one of a breathing rate parameter based on the subspace components is for identifying a breathing rate parameter based on a number of dominant subspace components being less than or equal to the determined threshold.

12. The apparatus as claimed in claim 11, wherein the means for generating, based on the identifying, at least one of a breathing rate parameter based on the subspace components is for:

bandpass filtering the dominant subspace components;
determining the bandpass filtered dominant subspace components have an energy value greater than an outage threshold value;
determining a peak motion event within the bandpass filtered dominant subspace components; and
mapping the number of peak motion events to a breathing rate parameter value.

13. The apparatus as claimed in claim 12, wherein the means for determining a peak motion event within the bandpass filtered dominant subspace components is for:

max-min normalising the dominant subspace components;
determining a minimum peak prominence (MINPRO), wherein the prominence of a peak determines how much the peak stands out, due to its height and location, relative to other peaks around it;
determining a minimum peak distance (MINDIST);
determining a minimum peak strength (MINSTR);
defining at least one peak motion event based on peaks which have a relative importance of at least MINPRO, and based on a MINDIST selected based on the human respiration rate ranging between 10 to 40 BPM and based on peaks of value greater than MINSTR times a median peak value.

14. The apparatus as claimed in any of claims 9 to 13, wherein the determined threshold is 1.

15. A method for determining motion of a user, the method comprising:

obtaining radio-frequency channel state information associated with a receiver;
analysing the radio-frequency channel state information to determine subspace components;
identifying from the subspace components a periodic breathing motion body motion from an other than breathing body motion;
generating, based on the identifying, at least one of a breathing rate parameter and a limb and/or body motion parameter based on the subspace components.

Figure 1

**Denoising Module** 3

**Body Movement Detector** 5

**Breathing Rate Monitor** 7

10 WiFi CSI Signal Input

1 First Order Difference

11 Lowpass Filter

13 PCA decomposition

15 Downsampling (Epoch) Construction

21 Multi-Dimensional Clustering-based Subspace Tracker

1 * PCA Projection

No body movements detected 60

31 Bandpass Filter

33 Outage Detector

Peak Detector 35

20 Breath Rate Signal Output

30 Body Movement Signal Output

40 Motion Signal Input

50 Breath rate Signal Input

Parameterization

Parameterization

Figure 2

```
┌─────────────────────────────────────────┐
│        Receive WiFi CSI Signal           │
│                                   101    │
└─────────────────────────────────────────┘
                    │
┌─────────────────────────────────────────┐
│       Determine first order difference   │
│                                   103    │
└─────────────────────────────────────────┘
                    │
┌─────────────────────────────────────────┐
│     Generate subspace representation     │
│        (Subspace processing)      105    │
└─────────────────────────────────────────┘
                    │
        ┌───────────┴───────────┐
┌──────────────────────┐   ┌──────────────────────┐
│  Obtain motion       │   │ Obtain breathing     │
│  estimates from      │   │ estimates from       │
│  motion subspace     │   │ breathing subspace   │
│  signals      107    │   │ signals       117    │
└──────────────────────┘   └──────────────────────┘
        │                          │
┌──────────────────────┐   ┌──────────────────────┐
│  Track motion        │   │  Track breathing     │
│  estimates           │   │  estimates           │
│             109      │   │              119     │
└──────────────────────┘   └──────────────────────┘
```

Figure 3

EP 3 692 898 A1

## Figure 4

```
┌─────────────────┐        ┌─────────────────┐        ┌─────────────────┐
│  Receive WiFi CSI│        │  Receive radar  │        │  Receive mat    │
│  signal          │        │  signals    303 │        │  signals    305 │
│             301  │        └────────┬────────┘        └────────┬────────┘
└────────┬────────┘                  │                          │
         │              ┌────────────┴──────────┐               │
         ▼              ▼                        ▼               │
┌─────────────────┐  ┌─────────────────┐  ┌─────────────────┐   │
│  Generate sub-  │  │  Train machine  │  │  Parameterization│  │
│  space          │  │  learning using │  │  of radar signals│  │
│  representation │  │  radar signals  │  │             306 │   │
│             302 │  │             304 │  └────────┬────────┘   │
└────────┬────────┘  └────────┬────────┘           │            │
```

**Receive WiFi CSI signal 301**

**Generate sub-space representation 302**

**Receive radar signals 303**

**Train machine learning using radar signals 304**

**Parameterization of radar signals 306**

**Receive mat signals 305**

**Obtain motion/breathing parameters 307**

**Use machine learning/AI based method trained on motion/breathing parameters and radar and mat signals to generate sleep parameters 309**

**Generate live sleep parameters based on trained system 311**

EP 3 692 898 A1

Figure 5

EP 3 692 898 A1

Figure 6

(a) motion false positives

(b) duration of motion false positives

(a) Outage rate

(b) small-scale outage duration

(c) large-scale outage duration

Figure 7

Europäisches
Patentamt

European
Patent Office

Office européen
des brevets

# EUROPEAN SEARCH REPORT

**Application Number**

EP 19 15 6321

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/156492 A1 (ORIGIN WIRELESS INC [US]) 14 September 2017 (2017-09-14) * paragraphs [0014], [0016], [0050], [0061], [0070], [0072], [0073], [0076], [0077], [0143] * * figure 8A * | 1-4,6-15 | INV. A61B5/0205 A61B5/08 A61B5/00 A61B5/113 A61B5/11 A61B5/0476 A61B5/0402 H04L1/06 |
| X | CN 108 553 108 A (YE WEI) 21 September 2018 (2018-09-21) * paragraphs [0014], [0027] * | 1,5,15 | |
| A | CN 106 936 526 A (UNIV NORTHWESTERN POLYTECHNICAL) 7 July 2017 (2017-07-07) * paragraphs [0012], [0020], [0031], [0032] * | 3,5 | ADD. A61B5/05 A61B5/0496 A61B5/0488 |
| A | US 2008/294019 A1 (TRAN BAO [US]) 27 November 2008 (2008-11-27) * paragraphs [0043], [0050] * | 4,7 | |
| A | US 2010/152600 A1 (DROITCOUR AMY [US] ET AL) 17 June 2010 (2010-06-17) * paragraph [0306] * | 12 | TECHNICAL FIELDS SEARCHED (IPC) A61B H04L |

The present search report has been drawn up for all claims

| Place of search | Date of completion of the search | Examiner |
|---|---|---|
| The Hague | 25 July 2019 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 15 6321

This annex lists the patent family members relating to the patent documents cited in  the above-mentioned European search report.
The members are as contained in the European Patent Office EDP file on
The European Patent Office is in no way liable for these particulars which are merely given for the purpose of information.

25-07-2019

| Patent document cited in search report | | Publication date | Patent family member(s) | | Publication date |
|---|---|---|---|---|---|
| WO 2017156492 | A1 | 14-09-2017 | CN | 108778106 A | 09-11-2018 |
| | | | EP | 3426137 A1 | 16-01-2019 |
| | | | JP | 2019512304 A | 16-05-2019 |
| | | | WO | 2017156492 A1 | 14-09-2017 |
| CN 108553108 | A | 21-09-2018 | NONE | | |
| CN 106936526 | A | 07-07-2017 | NONE | | |
| US 2008294019 | A1 | 27-11-2008 | US | 2008294019 A1 | 27-11-2008 |
| | | | US | 2010026479 A1 | 04-02-2010 |
| | | | US | 2014235965 A1 | 21-08-2014 |
| | | | US | 2017086672 A1 | 30-03-2017 |
| US 2010152600 | A1 | 17-06-2010 | US | 2010152600 A1 | 17-06-2010 |
| | | | US | 2010240999 A1 | 23-09-2010 |
| | | | US | 2010249630 A1 | 30-09-2010 |
| | | | US | 2010249633 A1 | 30-09-2010 |
| | | | US | 2010292568 A1 | 18-11-2010 |
| | | | WO | 2010132850 A1 | 18-11-2010 |

EPO FORM P0459

For more details about this annex : see Official Journal of the European Patent Office, No. 12/82